Europäisches Patentamt

European Patent Office

Office européen des brevets

(11) Veröffentlichungsnummer: **0 255 817**
**A2**

(12) **EUROPÄISCHE PATENTANMELDUNG**

(21) Anmeldenummer: 87890184.2

(22) Anmeldetag: 05.08.87

(51) Int. Cl.⁴: **G 01 N 27/22**
G 01 N 33/02

(30) Priorität: 06.08.86 AT 2123/86

(43) Veröffentlichungstag der Anmeldung:
10.02.88 Patentblatt 88/06

(84) Benannte Vertragsstaaten:
CH DE FR IT LI SE

(71) Anmelder: **MASCHINENFABRIK HEID
AKTIENGESELLSCHAFT
Ernstbrunner Strasse 31-33
A-2000 Stockerau (AT)**

**Schrack Elektronik-Aktiengesellschaft
Rudolfstrasse 189, Ecke Sonnleitnergasse 20
A-1100 Wien (AT)**

(72) Erfinder: **Kantilli, Günter
Pramergasse 3
A-1090 Wien (AT)**

**Kienmandl, Leopold
Columbusgasse 54/18
A-1100 Wien (AT)**

**Messner, Erich
Liechtensteinstrasse 61
A-1090 Wien (AT)**

(74) Vertreter: **Müllner, Erwin, Dr. et al
Patentanwälte Dr. Erwin Müllner Dipl.-Ing. Werner
Katschinka Postfach 159 Weihburggasse 9
A-1014 Wien (AT)**

(54) Vorrichtung zur Messung der relativen Feuchte von Erntegut in einem Trocknungsbehälter.

(57) Zur Bestimmung der Feuchtigkeit von Maiskolben über die Messung der Dielektrizitätskonstante wird vorgeschlagen, in einem Trocknungsbehälter eine senkrecht stehende Platten- sonde oder eine im wesentlichen waagrechte Seil- oder Stabsonde (4) vorzusehen. Es hat sich herausgestellt, daß die bekannten Sonden, nämlich senkrecht stehende, etwa 1/2 m lange Stabsonden, nur zur Bestimmung der Feuchtigkeit bei Mais- oder Getreidekörnern, nicht jedoch bei Maiskolben geeignet sind.

Fig. 1

EP 0 255 817 A2

# Beschreibung

"Vorrichtung zur Messung der relativen Feuchte von Erntegut in einem Trocknungsbehälter"

Die Erfindung betrifft eine Vorrichtung zur Messung der relativen Feuchte von Erntegut in einem Trocknungsbehälter mittels einer von der Umgebung isolierten, mit Hochfrequenz ansteuerbaren Sonde.

Bei bekannten Vorrichtungen dieser Art, die z.B. für Maiskörner verwendet werden, ist eine senkrecht stehende Stabsonde vorgesehen, die etwa 1/2 m in das zu messende Material hineinragt. Die Sonde wird mit einer Hochfrequenz angesteuert. Durch Strom-und Spannungsmessung kann auf die Dielektrizitätskonstante des Materials geschlossen werden, und von dieser auf die relative Feuchte.

Bei der Trocknung von Mais für die Saatgutherstellung wird der ganze Maiskolben getrocknet. Die Trocknung der Maiskolben erfolgt in Boxen verschiedener Abmessungen, in welchen ein Gitterrost eingezogen ist. Auf dem Gitterrost werden die Maiskolben geschüttet und mit Luft von unten nach oben bzw. umgekehrt durchströmt und so die Abtrocknung erreicht.

Versucht man, die oben beschriebene Meßmethode - die bei Maiskörnern brauchbare Ergebnisse liefert - bei ganzen Maiskolben anzuwenden, so stellt man fest, daß die Meßergebnisse sehr stark schwanken und kein Maß für die relative Feuchte der Maiskolben sind.

Man geht daher derzeit so vor, daß man die relative Feuchte der Maiskolben zu Beginn der Trocknung bestimmt und während des Trocknens die Temperatur und Feuchtigkeit der zu- und wegströmenden Luft mißt. Man kann dann (z.B. mittels eines Computerprogramms) berechnen, wieviel Feuchtigkeit durch die Luft abgeführt wird und so auf die relative Feuchte der Maiskolben schließen.

Dieses Verfahren ist jedoch zwangsläufig aufwendig und ungenau, weil die relative Feuchte der Maiskolben nicht direkt gemessen, sondern aus einer Vielzahl anderer Messungen errechnet wird.

Es ist Aufgabe der vorliegenden Erfindung, eine Vorrichtung zu schaffen, mit der auf einfache Weise eine genaue direkte Messung der relativen Feuchte von Maiskolben möglich ist.

Diese Aufgabe wird erfindungsgemäß dadurch gelöst, daß bei einer Vorrichtung der eingangs genannten Art die Sonde zur Messung der relativen Feuchte von Maiskolben als senkrecht stehende Plattensonde oder als im wesentlichen waagrechte Seil- und Stabsonde ausgebildet ist.

Es wurde überraschend gefunden, daß durch die erfindungsgemäße Ausbildung der Sonde auch bei Maiskolben eine auf 1 % genaue Messung der relativen Feuchte möglich ist.

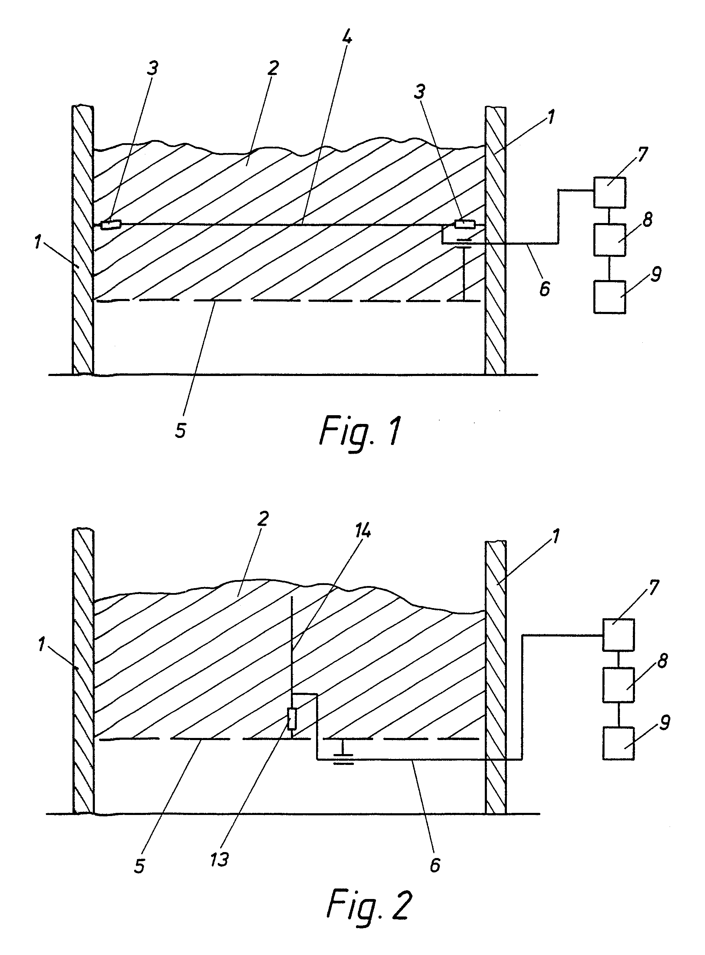

Anhand der beiliegenden Fig. 1 und 2 wird die Erfindung näher erläutert. Fig. 1 zeigt eine Vorrichtung mit einer Seilsonde und Fig. 2 eine Vorrichtung mit einer Plattensonde.

Der Trocknungsbehälter wird durch äußere Boxenbegrenzungen 1 gebildet. In diesem befindet sich ein waagrechter Metallgitterrost 5, auf dem die Maiskolben 2 liegen.

In dem Trocknungsbehälter ist gemäß Fig. 1 zwischen den Boxenbegrenzungen 1 eine Seilsonde 4 gespannt, die über Isolierstücke 3 mit den Boxenbegrenzungen 1 verbunden ist. Die Seilsonde 4 ist waagrecht angeordnet und erstreckt sich im wesentlichen über die gesamte Breite des Trocknungsbehälters. Anstelle einer Seilsonde kann auch eine (mechanisch stabilere) Stabsonde verwendet werden. Die Seil- bzw. Stabsonde 3 ist allseitig von Maiskolben 2 umgeben.

Gemäß Fig. 2 ist auf dem Metallgitterrost 5 eine senkrecht stehende Plattensonde 14 über ein Isolierstück 13 befestigt. Auch die Plattensonde 14 ist vollständig von Maiskolben 2 umgeben.

Die Sonde 4 bzw. 14 ist über eine Hochfrequenzleitung 6 mit einem Frequenzerzeuger 7 verbunden. Vom Hochfrequenzsender 7 wird zwischen der Sonde 4 bzw. 14 und dem Gitterrost 5 bzw. den äußeren Boxenzungen 1 ein hochfrequentes Feld aufgebaut und die Dielektrizitätskonstante der Maiskolben und somit ihre relative Feuchte gemessen. Über eine Auswerteinheit 8 mit Strom- oder Spannungsausgang kann die jeweilige Kolben-Feuchte auf einem Meßgerät 9 abgelesen werden oder einem Regelgerät zur Optimierung oder Steuerung des Trocknungsprozesses zugeführt werden oder automatische Abschaltungen des Trocknungsprozesses bei gewünschter Abtrocknung erzielt werden. Weiters ist eine Optimierung der Luft-und Wärmezufuhr über eine entsprechende Regelung möglich.

## Patentansprüche

Vorrichtung zur Messung der relativen Feuchte von Erntegut in einem Trocknungsbehälter mittels einer von der Umgebung isolierten, mit Hochfrequenz ansteuerbaren Sonde, dadurch gekennzeichnet, daß die Sonde zur Messung der relativen Feuchte von Maiskolben (2) als senkrecht stehende Plattensonde (14) oder als im wesentlichen waagrechte Seil- oder Stabsonde (4) ausgebildet ist.

0255817

Fig. 1

Fig. 2